# EUROPEAN PATENT APPLICATION

(11) **EP 1 961 440 A1**
(43) Date of publication of application: **27.08.2008**
(21) Application number: 06834891.1
(22) Date of filing: 18.12.2006
(51) Int. Cl.: A61M 16/04

(54) **TRACHEOTOMY TUBE AND METHOD OF USING THE SAME**

(30) Priority: 16.12.2005 JP 2005362962; 17.03.2006 JP 2006073744
(71) Applicant: Nomori, Hiroaki, Nakano-ku Tokyo 1640002 (JP)
(72) Inventor: Nomori, Hiroaki, Nakano-ku Tokyo 1640002 (JP)
(74) Representative: Setna, Rohan P.
(86) International application number: PCT/JP2006/325179
(87) International publication number: WO 2007/069766

(57) **Abstract**

To provide a tracheotomy tube, which is configured such that a balloon reliably inflates at the time of inhalation, that air is sent to the lungs without leaking, that exhaled air reaches the vocal cord when a patient wishes to vocalize, and that saliva is prevented from entering a trachea at the time of exhalation when the patient does not wish to vocalize. The balloon keeps its inflated shape under normal pressure, and when air is sent from an artificial respiration device to the balloon via a hole provided in an inside tube portion, the balloon substantially maintains a state of being bonded to a trachea and maintains the inflated shape under pressure of exhaled air that is not for vocalization, and the inflated shape contracts under pressure of exhaled air for vocalization, and the exhaled air is sent toward the vocal cord via a gap formed between the balloon having the contracted shape and a tracheal wall.

## Description

### BACKGROUND

The present invention relates to a tracheotomy tube, and particularly to a tracheotomy tube that allows more specifically speech when exhaling air from the lungs even when cutting open a trachea to insert the tracheotomy tube into the body, and also relates to a method of using the tracheotomy tube.

Artificial breathing using a regular tracheotomy tube is performed by cutting open a trachea, connecting a tube having a balloon is inserted into the trachea, and air is sent into the balloon, the balloon is press-bonded to an internal wall of the trachea, and oxygen or air is fed to the lungs by means of a artificial respiration device so that the oxygen or air does not flow into the mouth.

In the case of this method, the balloon is constantly inflated to block the gap between the trachea and the tube, whereby the air inhaled and exhaled reciprocates between the artificial respiration device and the lungs. Otherwise, when air is sent, a large volume of air leaks from the mouth without reaching the lung, whereby effective ventilation cannot be performed.

On the other hand, a vocal cord is positioned between the trachea and the mouth, thus a conventional tracheotomy tube cannot sent air to the vocal cord, and therefore a patient cannot vocalize. Therefore, in order to solve this problem, the applicant of the present application has proposed a tracheotomy tube that allows speech even if the tracheotomy tube with a balloon is inserted into a trachea (Japanese Published Unexamined Patent Application No. 2003-210585). This tracheotomy tube has an inside tube portion disposed within a trachea, an outside tube portion connected to a artificial respiration device, and a balloon disposed on the outer circumference of the inside tube portion, wherein the balloon is attached to the outside of the inside tube portion so that the inside and outside of the balloon cannot communicate with each other, and the inside tube portion has a hole for communicating the inside of the inside tube portion with the inside of the balloon.

According to this tracheotomy tube, at the time of inhalation, that is, when the artificial respiration device feeds air, air is fed from the artificial respiration device into the inside of the balloon via the hole provided in the inside tube portion, whereupon the balloon inflates to block the gap between the trachea and tube so that the air fed by the artificial respiration device flows into the lungs without leaking. At the time of exhalation, that is, when exhaling, the air on the inside of the balloon returns to the inside of the inside tube portion via the hole provided in the inside tube portion, whereupon the balloon shrinks to its original size so as to contact with the external wall of the inside tube portion, whereby a gap is formed between the tube and the trachea and some of the exhaled breath flows out to the outside of the body via the vocal cord. Consequently, the patient can vocalize.
Patent Literature 1: Japanese Published Unexamined Patent Application No. 2003-210585

### SUMMARY

With the conventional tracheotomy tube, if the balloon does not inflate completely at the time of inhalation, then the air fed from the artificial respiration device leaks from the gap between the balloon and a tube wall, and therefore the patient cannot be ventilated, and at each exhalation the air spreads over the vocal cord, imposing a burden on the patient, and at the time of exhalation saliva spreads over the area ahead of the balloon via the space between the trachea and the shrunk balloon.

Therefore, in order to solve such problems, it is an object of the present invention to provide a tracheotomy tube, which reliably inflates a balloon at the time of inhalation to feed air into the lungs without causing air leakage, and allows exhaled air to spread over the vocal cord when the patient wishes to vocalize but prevents saliva from entering the trachea at the time of exhalation when the patient does not wish to vocalize.

In order to achieve the above object, the present invention is a tracheotomy tube, which has an inside tube portion to be disposed within a trachea, an outside tube portion to be connected to an artificial respiration device, and a balloon disposed on an outer circumference of the inside tube portion, the balloon being attached to the outside of the inside tube portion so that the inside and outside of the balloon cannot communicate with each other, and the inside tube portion having a hole for communicating the inside of the inside tube portion with the inside of the balloon, wherein the balloon keeps the inflated shape at normal pressures, when the artificial respiration device feeds air into the balloon via the hole provided in the inside tube portion the balloon substantially maintains a state in which the balloon adheres to the trachea, and at the same time the balloon firmly adheres to a wall of the trachea as the pressure of the balloon increases, whereby the air is fed into the lungs without leaking, and the balloon keeps the inflated shape at the pressure of exhaled air when a patient does not wish to vocalize, but the inflated shape shrinks at the pressure of the exhaled air when the patient wishes to vocalize, and the exhaled air is fed toward the vocal cord via the gap between the shrunk balloon and the wall of the trachea.

In a preferred embodiment of the present invention, at least one of the material, size, shape and thickness of the balloon is adjusted so that the inflated shape is maintained at the pressure of inhaled air and the pressure of exhaled air when the patient does not wish to vocalize, and the inflated shape shrinks at the pressure of the exhaled air when the patient wishes to vocalize.

The tracheotomy tube of the present invention can be manufactured using a known method such as compression molding and dip molding (see Japanese Published Unexamined Patent Application No. 2004-290366, Japanese Examined Patent Publication No. H7-4427). In a preferred method of producing the tracheotomy tube of the present invention, a dipping method is used in which a spherical, oval, or trapezoidal mold is immersed in liquid made from silicon rubber, urethane, or other material capable of freely changing its shape, whereby a film of silicon or the like is formed around the spherical mold, and thereafter the immersed spherical mold is taken out as a balloon, and the mold is removed from an opening formed on each end of the balloon, and then an inside tube is inserted into this opening to seal a connecting portion between the inside tube and the balloon. Conventionally a cylindrical tube was used, but use of this cylindrical tube creates a shrunk balloon due to normal pressure. For this reason, it is preferred to use a spherical or oval mold.

Examples of the preferred material of the balloon include silicon rubber, urethane, vinyl chloride, natural rubber, thermoplastic elastomer, and other elastic material capable of changing its shape, and the preferred film thickness is 0.01 through 0.7 mm, preferably 0.05 through 0.5 mm, and more preferably 0.1 through 0.3 mm. Accordingly, the spherical state of the balloon can be maintained under normal pressures. However, a thin film made of silicon or the like shrinks under low pressure. If the film thickness is less than 0.01 mm, the inflated shape is caused to contract by exhaled breath for vocalizing, and if the film thickness exceeds 0.7 mm, the inflated shape may not be caused to contract by exhaled breath for vocalizing. The hardness of the balloon is preferably 20 through 60 or more preferably 40 on the basis of JIS K6301 (shape A), and the degree of extension thereof is preferably 200 through 1500 % on the basis of JIS K6301 (No. 3). The hardness of the balloon is appropriately adjusted by thermal plastics.

It is preferred that the balloon be formed into a circular shape so as to be able to block air leakage from the respiration device around the entire circumference with respect to the cross section of the air passage (radial direction cross section). The diameter of the balloon is 20 through 30 mm when it is inflated. It is preferred that the balloon have a shape (circular shape, oval shape, rectangular round shape) with respect to the axial direction of the air passage (longitudinal direction) so as to be able to cover a situation where the balloon is placed in the air passage and thereby mispositioning occurs. The diameter of the balloon is 20 through 30 mm. It is preferred that the shape of the balloon be spherical shape, rectangular round shape, or other substantially spherical shape. Although the balloon is in the shape of a sphere, preferably the balloon has an oval shape having slightly a large diameter in a direction perpendicular to a direction in which the bronchial tube extends (1/3 at most with respect to the direction in which the bronchial tube extends). If the balloon has this shape, the balloon can keep the spherical shape thereof under the normal pressure or the pressure of the exhaled air that is not generated by vocalization, and at the same time the contact area with the airway wall can be made small, thus the balloon can be caused to shrink by the pressure of the exhaled air that is generated when vocalization, whereby air easily passes through between the trachea and the balloon.

### DESCRIPTION OF THE DRAWINGS

Fig. 1 shows a schematic view of a tracheotomy tube disposed in the trachea of a patient.
Fig. 2 is a partially enlarged perspective view of an inside tube portion having a balloon.
Fig. 3 is a schematic view of the tracheotomy tube showing a state of exhaled air that is not for vocalization.
Fig. 4 is a schematic view of the tracheotomy tube showing a state of exhaled air that is for vocalization.

### DETAILED DESCRIPTION

As described above, the inventor of the present invention has focused attention on the difference between the pressure of exhaled air for vocalization and the pressure of exhaled air that is not for vocalization in a patient attached with a tracheotomy tube, and provides a tracheotomy tube having a balloon that shrinks under the former pressure but keeps the inflated shape thereof under the latter pressure, whereby when the exhaled air reaches the vocal cord when the patient wishes to vocalize and when the exhaled air without voice is obtained, saliva is prevented from entering the trachea.

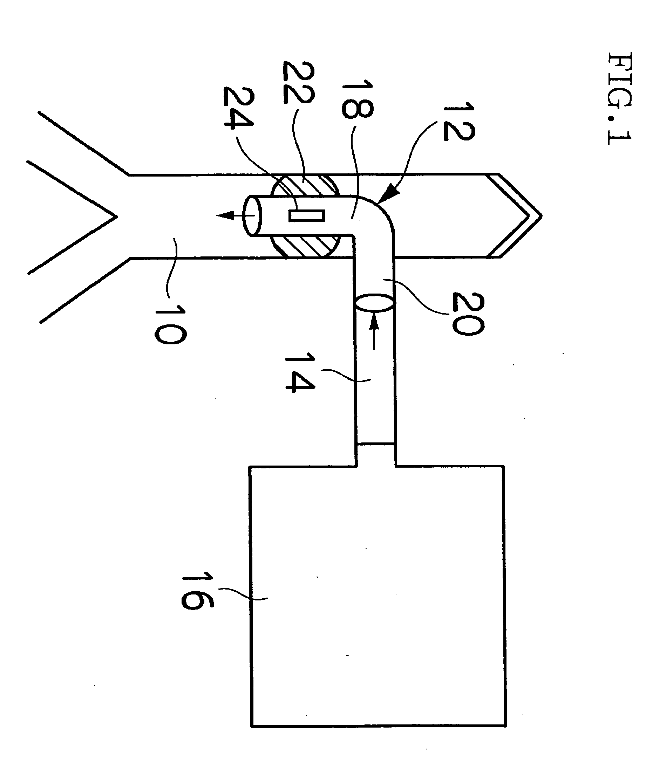

Fig. 1 shows a tracheotomy tube 12 that is disposed within the trachea 10 of a patient. Fig. 1 shows a state in which air is inspired. This tracheotomy tube 12 is connected to an artificial respiration device 16 via a communicating tube 14. The tracheotomy tube 12 has an inside tube portion 18 disposed within the trachea, an outside tube portion 20 connected to the artificial respiration device 16, and a balloon 22 disposed on the entire circumference of the inside tube portion.

The balloon 22 is attached to the external wall of the inside tube portion 18 by means of an adhesive or the like throughout the entire circumference of the inside tube portion 18 at, for example, upper and lower portions of the balloon 22 so that the inside and the outside of the balloon 22 are not communicated to each other. The inside tube portion 18 has a hole 24 that allows communication between the inside of the inside tube portion 18 and the inside of the balloon 22. This hole 24 is configured by holes of various shapes, opening portion shaving slit, mesh or columnar structure, and the like.

The artificial respiration device 16 repeats inhalation and exhalation on a predetermined cycle, wherein at the time of inhalation a predetermined amount of oxygen or air is discharged at a predetermined pressure, and at the time of exhalation the inside of the tracheotomy tube 12 and the atmosphere are caused to communicate to each other. When the patient attached with the tracheotomy tube exhales on his/her own, he/she exhales somewhat strongly when intending to vocalize, and exhales weakly when not intending to vocalize. In the former case the expiratory pressure is normally 5 through 10 cmHg, while in the latter case the expiratory pressure is normally 2 through 3 cmHg.

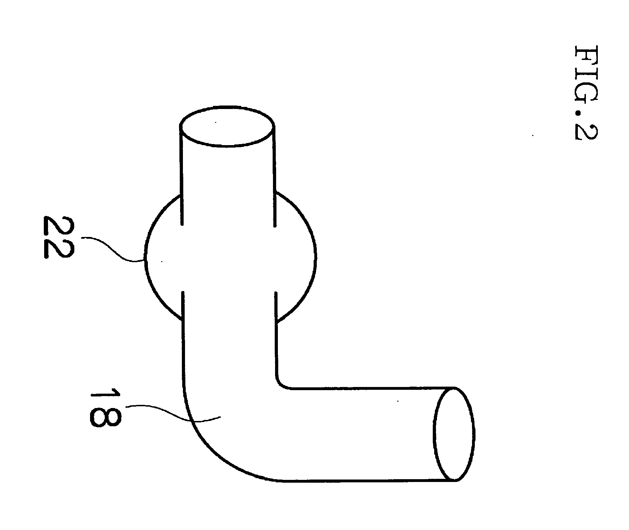

Fig. 2 is a partially enlarged perspective view of the inside tube portion 18 provided in the balloon 22. The balloon is formed from a silicon thin film, as described above, and keeps its substantially spherical shape under normal pressure. When this balloon is inserted into the trachea and air is supplied from the artificial respiration device to the balloon at a pressure of 5 through 100 cmHg, the balloon 22 further inflates and is thereby fixedly bonded to the tracheal wall as shown in Fig. 1. Specifically, at the time of inhalation, when oxygen or air is fed into the tracheotomy tube 12 from the artificial respiration device 16, the air is fed into the inside of the balloon 22 via the hole 24 of the inside tube portion 18, the balloon 22 that is in the inflated form under normal pressure further inflates, and consequently the external surface of the balloon 22 is strongly and fixedly bonded to a side surface of an air trachea 10, whereby the communication between the trachea on the lung side and the trachea on the mouth side is prevented. Accordingly, oxygen or air can be fed into the lungs of the patient effectively without causing air leakage.

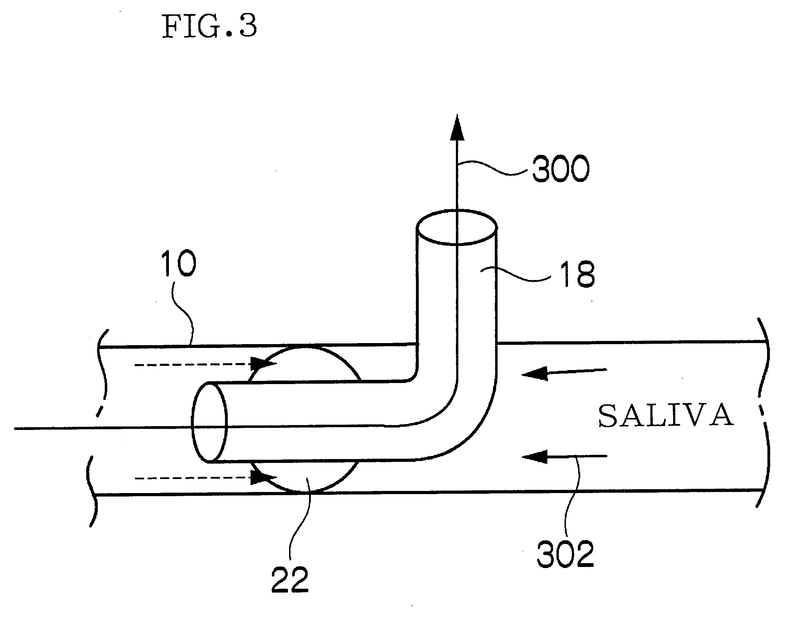

Fig. 3 shows a state of exhaled air for vocalization. At this moment, the pressure of the exhaled air is comparatively weak, thus the inflated shape of the balloon 22 is not contracted by the expiratory pressure and keeps its substantially the original shape. Since the balloon is in contact with the internal wall of the trachea 10, no gap is formed between the balloon and the internal wall of the trachea, whereby exhaled air 300 enters the inside tube 18. Since the balloon 22 is in contact with the internal wall of the trachea, saliva 302 does not enter the trachea on the lung side.

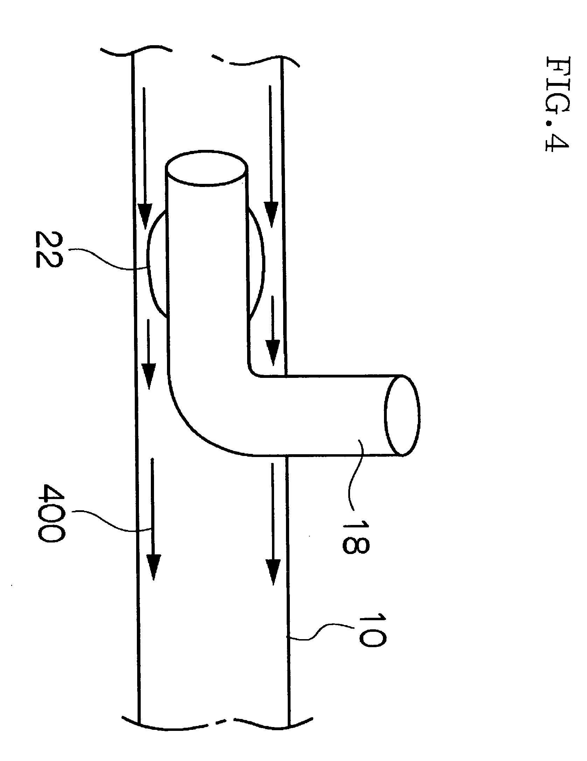

Fig. 4 shows a state of exhaled air that is not for vocalization. At this moment, the pressure of the exhaled air is comparatively strong, thus the inflated shape of the balloon is shrunk by the exhaled air, whereby a gap through which the exhaled air can pass is formed between balloon 22 and the trachea 10. When the exhaled air 400 passes through this gap and reaches the vocal cord, vocalization becomes possible. Moreover, at the time of vocalization, the pressure generated thereupon discharges saliva accumulated on the mouth side of the balloon to the mouth side.

By appropriately adjusting at least one of the material, shape, film thickness and size of the balloon, it is possible to realize contraction of the circular shape of the balloon, which is formed under normal pressure, by the exhaled air that is for vocalization, and maintenance of the circular shape without being shrunk when air is exhaled without intending to vocalize.

As one example of the material of the balloon, there is silicon rubber. The hardness of the silicon rubber is preferably 20 through 60 or more preferably 40 on the basis of JIS K6301 (shape A), and the degree of extension thereof is preferably 200 through 1500 % on the basis of JIS K6301 (No. 3). Furthermore, the silicon rubber preferably has strong tear strength and has own adhesiveness.

First Production Example 60 g of a commercially available strip-shaped silicon rubber was weighed and placed in a 500-cc beaker. This silicon rubber was slowly stirred while adding toluene a little bit at a time to obtain a toluene dispersion liquid, and a 100-mm Φ aluminum round bar mold, which was previously applied with mold release agent and then air-dried, was immersed in the toluene dispersion liquid by lifting it up and down several times, whereby a uniform coating film was created. This coating film was aid-dried sufficiently, and thereafter subjected to heat hardening in an oven heated to 150°C for 10 through 15 minutes. Next, the balloon was released from the mold and washed in water. The balloon was attached to a silicon-rubber endotracheal tube (external diameter thereof is 10.4 mm) to form the tracheotomy tube of the present invention. The thickness of the balloon was 0.2 mm.

Second Production Example (compression molding) The balloon was formed into a spherical shape, the diameter of a balloon mold was 30 mm, the thickness of the balloon was an average of 0.1 mm, a silicon rubber was used as the material of the balloon, and the grade thereof was Silastic (product name) QF-4840 (Dow Corning Co., Ltd.). The material was placed in top and bottom molds, was subjected to primary vulcanization at 190°C for 15 minutes, thereafter was subjected to secondary vulcanization at 190°C for 5 minutes, and then the balloon was removed from the molds.

Third Production Example (dip molding) The balloon was formed into a spherical shape, the diameter of a balloon mold was 30 mm, the thickness of the balloon was an average of 0.1 mm, a silicon rubber was used as the material of the balloon, and the grade thereof was Silastic (product name) QF-4720 (Dow Corning Co., Ltd.). The material is subjected to viscosity adjustment using an appropriate amount of toluene. The viscosity at this moment is preferably 700 mpa-s. After molding a balloon having a diameter of 30 mm, the balloon was immersed in a created solution, the material was applied on the surface of the mold to form a thin film, and then the balloon was pulled out. For the purpose of preventing thickness irregularity, the forming die was rotated at 100 rpm for 5 minutes. These processes of immersion, pulling up, and rotation were repeated seven times. Thereafter, the balloon was subjected to heating and vulcanization in an oven heated to 120°C for 4 hours. The balloon was cooled in room temperature and then removed from the forming die.

Next, this tracheotomy tube was applied to cases who require artificial respiration. There were three cases: 80-year old man (COPD); 71-year old man (aftereffect of tuberculosis); and 83-year old man (COPD), who were supervised with their artificial respiration.

The mode of the artificial respiration devices of two cases was "pressure control" and that of one case was "volume control." Previously, the three were not able to communicate because they were supervised with the conventional tracheotomy tubes, but after replacing them with the tracheotomy tubes of the present invention (production examples 1, 2, 3) the three cases were able to communicate when exhaling. Since the three were able to communicate after the replacement with the tracheotomy tubes of the present invention, their QOLs were improved dramatically. In all of the three cases, air leakage at the time of inhalation and symptoms such as aspiration pneumonia were not observed during approximately one-month median follow-up. Moreover, each of the tubes described in the production examples 1 through 3 were inserted into a model trachea having a diameter of 23 mm by using IMI test back R5 (produced by IMI Co., Ltd.) and all-purpose ventilator LP10 (artificial lung (produced by Nellcor Puritan Bennett Inc.)), to carry out an experiment on exhaled air and inhaled air, and as a result, the balloon having an inflated shape under normal pressure kept bonded to the tracheal wall under the pressure of exhaled air that is not for vocalization, but a gap was formed between the balloon having an inflated shape under normal pressure and the tracheal wall under the pressure of exhaled air that is for vocalization, thus it was confirmed that the air from the artificial lung reaches an end side of the tube that is opposite to the model lung from the balloon.

According to the embodiment described above, although silicon rubber was used as the material of the balloon, an elastic material such as urethane or vinyl chloride can be used. Furthermore, the balloon can be integrally formed on the inside tube. Moreover, in the above-described embodiment, although the balloon was produced by means of a dipping method or a compression molding method, the balloon can be molded using a metallic split mold and thus obtained balloon can be adhered to a tube to obtain the tracheotomy tube of the present invention. Moreover, the balloon and the tube can be molded integrally.

### DESCRIPTION OF REFERENCE NUMERALS

Trachea of patient: 10, tracheotomy tube: 12, communicating tube: 14, artificial aspiration device: 16, inside tube portion disposed within trachea: 18, outside tube portion: 20, balloon: 22

## Claims

1. A tracheotomy tube, comprising:
an inside tube portion to be disposed within a trachea;
an outside tube portion to be connected to an artificial respiration device; and
a balloon disposed on an entire outer circumference of the inside tube portion, the balloon being attached to the outside of the inside tube portion so that the inside and outside of the balloon cannot communicate with each other, and the inside tube portion having a hole that allows communication between the inside of the inside tube portion and the inside of the balloon, wherein
the balloon keeps an inflated shape thereof under normal pressure, and when the artificial respiration device sends air to the balloon via the hole provided in the inside tube portion, the balloon substantially maintains a state of being bonded to the trachea, maintains the inflated shape under pressure of exhaled air that is not for vocalization, and contracts the inflated shape thereof under pressure of exhaled air for vocalization, and the exhaled air is sent toward a vocal cord via a gap formed between the contracted balloon and a tracheal wall.

2. The tracheotomy tube according to claim 1, wherein the balloon has a film thickness of 0.01 through 0.7 mm, is molded so as to keep the inflated shape under normal pressure, maintains the inflated shape thereof when the pressure of the exhaled air that is not for vocalization is 2 through 3 cmHg, and contracts the inflated shape thereof when the pressure of the exhaled air for vocalization is 5 through 10 mmHg, and the exhaled air for vocalization is sent toward the vocal cord.

3. A method of using a tracheotomy tube having: an inside tube portion to be disposed within a trachea; an outside tube portion to be connected to an artificial respiration device; and a balloon disposed on an entire outer circumference of the inside tube portion, the balloon being attached to the outside of the inside tube portion so that the inside and outside of the balloon cannot communicate with each other, and the inside tube portion having a hole that allows communication between the inside of the inside tube portion and the inside of the balloon, wherein
the balloon keeps an inflated shape thereof under normal pressure, and when the artificial respiration device sends air to the balloon via the hole provided in the inside tube portion, the balloon substantially maintains a state of being bonded to the trachea, maintains the inflated shape under pressure of exhaled air that is not for vocalization, and contracts the inflated shape under pressure of exhaled air for vocalization, and the exhaled air is sent toward a vocal cord via a gap formed between the contracted balloon and a tracheal wall.

## Amended claims

### Amended claims under Art. 19.1 PCT

**1.** (Amended) A tracheotomy tube, comprising:
an inside tube portion to be disposed within a trachea;
an outside tube portion to be connected to an artificial respiration device; and
a balloon disposed on an entire outer circumference of the inside tube portion, the balloon being attached to the outside of the inside tube portion so that the inside and outside of the balloon cannot communicate with each other, and the inside tube portion having a hole that allows communication between the inside of the inside tube portion and the inside of the balloon, wherein
an entire shape of the balloon keeps an inflated shape under normal pressure, and when the artificial respiration device sends air to the balloon via the hole provided in the inside tube portion, the balloon substantially maintains a state of being bonded to the trachea, and maintains the inflated shape under pressure of exhaled air that is not for vocalization, and the entire shape of the balloon contracts from the inflated shape under pressure of exhaled air for vocalization, and the exhaled air is sent toward a vocal cord via a gap formed between the entire shape of the contracted balloon and a tracheal wall.

**2.** The tracheotomy tube according to claim 1, wherein the balloon has a film thickness of 0.01 through 0.7 mm, is molded so as to keep the inflated shape under normal pressure, maintains the inflated shape thereof when the pressure of the exhaled air that is not for vocalization is 2 through 3 cmHg, and contracts the inflated shape when the pressure of the exhaled air for vocalization is 5 through 10 mmHg, and the exhaled air for vocalization is sent toward the vocal cord.

**3.** A method of using a tracheotomy tube having: an inside tube portion top be disposed within a trachea; an outside tube portion to be connected to an artificial respiration device; and a balloon disposed on an entire outer circumference of the inside tube portion, the balloon being attached to the outside of the inside tube portion so that the inside and outside of the balloon cannot communicate with each other, and the inside tube portion having a hole that allows communication between the inside of the inside tube portion and the inside of the balloon, wherein
the balloon keeps an inflated shape thereof under normal pressure, and when the artificial respiration device sends air to the balloon via the hole provided in the inside tube portion, the balloon substantially maintains a state of being bonded to the trachea, maintains the inflated shape under pressure of exhaled air that is not for vocalization, and contracts the inflated shape under pressure of exhaled air for vocalization, and the exhaled air is sent toward a vocal cord via a gap formed between the contracted balloon and a tracheal wall.

**4.** (Added) The tracheotomy tube according to claim 1 or 2, wherein the inflated shape of the balloon is a spherical shape or an oval shape.

**5.** (Added) The tracheotomy tube according to claim 1 or 2, wherein the balloon has an oval shape having a large diameter in a direction perpendicular to a direction in which the bronchial tube extends.
